# EUROPÄISCHE PATENTANMELDUNG

(11) **EP 2 952 110 A1**
(43) Veröffentlichungstag der Anmeldung: **09.12.2015**
(21) Anmeldenummer: 15166823.3
(22) Anmeldetag: 07.05.2015
(51) Int. Cl.: A24F 47/00, A61M 15/06, A61M 11/04, A61M 15/00

(54) **ELEKTRISCHES INHALATIONSGERÄT, SPEICHERELEMENT FÜR EIN ELEKTRISCHES INHALATIONSGERÄT SOWIE VERFAHREN ZUM BEREITSTELLEN EINES SOLCHEN**

(30) Priorität: 06.06.2014 DE 102014210964
(71) Anmelder: Klocke Verpackungs-Service GmbH, 76356 Weingarten (DE)
(72) Erfinder: Klocke, Carsten, 76133 Karlsruhe (DE)
(74) Vertreter: Lemcke, Brommer & Partner Patentanwälte Partnerschaft mbB

(57) **Zusammenfassung**

Es wird ein Speicherelement für in einem elektrischen Inhalationsgerät, insbesondere in einer elektrischen Zigarette zu verdampfende Flüssigkeiten 2 vorgeschlagen, welches im Wesentlichen aus einem Material 4 besteht, das mit Flüssigkeit 2 getränkt werden kann, und als auswechselbare, separat handhabbare Einheit ausgebildet ist. Vorzugsweise ist das Speicherelement in einem Teilbereich seiner Oberfläche mit einer flüssigkeitsundurchlässigen Hülle 5 versehen.

Ferner wird eine elektrische Zigarette mit einem solchen Speicherelement vorgeschlagen, sowie ein Verfahren zum Bereitstellen eines Speicherelements, wobei das Speicherelement in ein flüssigkeits- und dampfdichtes Behältnis eingelegt, die Flüssigkeit in das Behältnis dosiert und das Behältnis dann geschlossen wird, so dass das Tränken des tränkbaren Materials mit zu verdampfender Flüssigkeit wenigstens zum Teil im geschlossenen Behälter erfolgt.

## Beschreibung

Die vorliegende Erfindung betrifft ein Speicherelement für in einem elektrischen Inhalationsgerät, insbesondere in einer elektrischen Zigarette zu verdampfende Flüssigkeiten, ein Verfahren zum Bereitstellen eines solchen Speicherelements sowie ein elektrisches Inhalationsgerät, insbesondere eine elektrische Zigarette mit einem solchen Speicherelement.

Elektrische Zigaretten, die mitunter auch elektronische Zigaretten oder kurz E-Zigaretten genannt werden, werden als Alternative zu Tabakzigaretten und Tabakpfeifen verwendet, um dem Anwender zu ermöglichen, pharmazeutisch wirksame Wirkstoffe, insbesondere Nikotin, wie beim Rauchen von Tabak zu inhalieren, ohne die Umgebung durch Rauch zu beeinträchtigen. Dementsprechend weisen elektrische Zigaretten normalerweise eine Form auf, die herkömmlichen Tabakzigaretten im weitesten Sinne ähnelt: Ein stabförmiges Gebilde, an dessen einen Ende ein Mundstück angebracht ist, an dem der Anwender saugen kann, um die Wirkstoffe zu inhalieren.

Elektrische Inhalationsgeräte, insbesondere E- Zigaretten umfassen in der Regel eine elektrische Energiequelle, einen Verdampfer für Flüssigkeiten, ein Speicherelement für die zu verdampfenden Flüssigkeiten und ein Mundstück. Die elektrische Energiequelle besteht normalerweise aus einer Batterie bzw. einem wiederaufladbaren Akkumulator und versorgt den Verdampfer mit elektrischer Energie. Der Verdampfer erwärmt die zu verdampfende Flüssigkeit auf Temperaturen von typischerweise zwischen 65°C und 120°C und ist meist luftdurchlässig ausgestaltet, so dass Luft durch ihn hindurch in das Mundstück und aus diesem heraus gesogen werden kann, um die verdampfte Flüssigkeit gasförmig oder in Form eines fein verteilten Tröpfchennebels zu inhalieren. Um bei einer elektrischen Zigarette zu gewährleisten, dass nur dann Flüssigkeit verdampft wird, wenn der Anwender am Mundstück saugt, wird die elektrische Energieversorgung für den Verdampfer mittels eines manuellen Schalters an der elektrischen Zigarette oder getriggert durch den Saugvorgang ein- und ausgeschaltet. Um für den Verdampfer einen Bereich in der elektrischen Zigarette zu schaffen, der beim Saugen am Mundstück von Luft durchströmt wird, befinden sich üblicherweise stromaufwärts des Verdampfers Luftzutrittsöffnungen im Gehäuse der elektrischen Zigarette.

Das Speicherelement eines elektrischen Inhalationsgeräts der vorliegenden Art sitzt in der Nähe des Verdampfers, da es diesen mit der zu verdampfenden Flüssigkeit versorgt. Es besteht meist aus einem nachfüllbaren Flüssigkeitstank oder einer auswechselbaren Kartusche, also einem Flüssigkeitsgefäß, dessen Öffnung mit beispielsweise einer leicht durchstoßbaren Folie verschlossen ist; diese wird beim Einsetzen der Kartusche in das Inhalationsgerät von einer entsprechend stabil ausgestalteten Leitung zum Verdampfer durchstoßen und somit geöffnet. Daneben werden bei elektrischen Zigaretten auch Schwämmchen als Speicherelemente verwendet, d.h. diese Speicherelemente bestehen aus einem Material, das mit Flüssigkeit getränkt werden kann. Solche Schwämmchen sind fest mit dem Mundstück der elektrischen Zigarette verbunden und werden mit diesem ausgewechselt, wenn das Speicherelement keine Flüssigkeit mehr abgeben kann; es handelt sich also um Verbrauchsmaterial.

Die zu verdampfenden Flüssigkeiten für elektrische Zigaretten enthalten in der Regel Nikotin als pharmazeutisch wirksamen Stoff in unterschiedlichen Konzentrationen. Daneben sind die verschiedensten Aromastoffe verwendbar, die dem Anwender einen sehr individuellen Genuss ermöglichen, bis hin zu einem nikotinfreien Inhalieren. Übliche Flüssigkeiten bestehen im Wesentlichen aus Propylenglycol- und Glyzerinlösungen, die als Vernebelungsmittel und Trägersubstanzen für die weiteren Inhaltsstoffe wie Aromen und Nikotin dienen. Die Nikotinkonzentrationen von am Markt erhältlichen Flüssigkeiten liegen in der Regel zwischen 0 bis 18 mg/ml, in Einzelfällen auch darüber. Als Aromen und Geschmacksstoffe werden beispielsweise Vanilleextrakt, Menthol, Apfelsäure, Ethylacetat und Linalool sowie auch Aromen verwendet, die für herkömmliche Tabakerzeugnisse charakteristisch sind.

Das Wiederbefüllen von Tanks in entsprechend ausgestatteten elektrischen Zigaretten ist insbesondere dann nicht unproblematisch, wenn - was die Regel ist - Nikotin enthaltende Flüssigkeiten verwendet werden. Denn Nikotin wird auch über die Haut in den menschlichen Organismus aufgenommen, was bei den vorliegenden Konzentrationen schon bei einem Kontakt mit wenigen Millilitern Flüssigkeit aufgrund der toxischen Wirkung des Nikotins bedenklich sein kann.

Kartuschen mit durchstoßbar verschlossenen Öffnungen verringern die Gefahr eines Hautkontakts mit den Flüssigkeiten beim Wiederbeladen der elektrischen Zigarette, schließen diesen jedoch nicht aus und bringen darüber hinaus einen hohen Materialverbrauch mit sich, da die Kartusche in der Regel aus Kunststoff spritzgegossen wird und mit einer leicht durchstoßbaren Folie, in der Regel aus Aluminium, versehen werden muss. Die Kartusche wird nach Gebrauch weggeworfen.

Elektrische Zigaretten, deren Mundstücke mit einem getränkten Schwämmchen als Speicherelement versehen sind, vermeiden zwar das Problem einer Kontaminierung der Finger mit Flüssigkeit beim Beladen der elektrischen Zigarette, jedoch wird das Mundstück hierdurch zum Wegwerfartikel, da es nach Gebrauch entsorgt werden muss. Hier ist der Materialverbrauch besonders hoch, da das Mundstück aus hochwertigem und widerstandsfähigem Kunststoff spritzgegossen wird, um dem Anwender eine hochwertige Anmutung und ein angenehmes Gefühl bei der Anwendung zu vermitteln.

Der vorliegenden Erfindung liegt daher die Aufgabe zugrunde, ein Speicherelement für ein elektrisches Inhalationsgerät, insbesondere für eine elektrische Zigarette, ein Verfahren zum Bereitstellen eines solchen Speicherelements sowie ein elektrischs Inhalationsgerät mit einem solchen Speicherelement vorzuschlagen, mit welchen die Handhabung beim Beladen des Inhalationsgeräts, insbesondere einer elektrischen Zigarette mit Wirkstoff vereinfacht und der Materialverbrauch unter Kosten- und Umweltaspekten reduziert ist.

Gelöst ist diese Aufgabe durch ein Speicherelement mit den Merkmalen des Patentanspruchs 1, durch ein Verfahren mit den Merkmalen des Patentanspruchs 5 sowie durch ein elektrisches Inhalationsgerät mit den Merkmalen des Patentanspruchs 8. Bevorzugte Ausgestaltungen und Weiterbildungen des erfindungsgemäßen Speicherelements finden sich in den Ansprüchen 2 bis 4; vorteilhafte Weiterbildungen des erfindungsgemäßen Verfahrens sind in den Patentansprüchen 6 und 7 niedergelegt; bevorzugte Ausgestaltungen des erfindungsgemäßen elektrischen Inhalationsgeräts sind in den Ansprüchen 9 und 10 definiert.

Gemäß der vorliegenden Erfindung wird also ein Speicherelement für die in einem elektrischen Inhalationsgerät, insbesondere in einer elektrischen Zigarette zu verdampfenden Flüssigkeiten vorgeschlagen, das im Wesentlichen aus einem Material besteht, das mit Flüssigkeit getränkt werden kann, und als auswechselbare, separat handhabbare Einheit ausgebildet ist. Dadurch, dass das erfindungsgemäße Speicherelement als auswechselbare Einheit ausgebildet ist, entfällt jegliches Nachfüllen des Speicherelements, so dass die Gefahr einer Kontaminierung der Finger des Anwenders mit Nikotin enthaltenden oder sonstigen pharmazeutischen Wirkstoffe enthaltenden Flüssigkeiten eliminiert ist. Gleichwohl ist der Materialverbrauch und der Entsorgungsaufwand für die erfindungsgemäßen Speicherelemente minimal, da keine aufwändigen oder hochwertigen, in der Regel spritzgegossenen Teile hergestellt und entsorgt werden müssen. Vielmehr können die erfindungsgemäßen Speicherelemente komplett aus leicht verfügbaren und als Verbrauchsmaterial geläufigen Materialien bestehen, wie beispielsweise Zellulosefasern oder Baumwollfasern, die darüber hinaus den Vorteil haben, aus nachwachsenden Rohstoffen gefertigt und kompostierbar zu sein.

Erfindungsgemäße Speicherelemente können fertig mit flüssigem Wirkstoff getränkt vertrieben werden, was eine Rückverfolgbarkeit gewährleistet; dies ist im Hinblick darauf, dass es sich insbesondere bei Nikotin um einen pharmazeutisch wirksamen Stoff handelt, der sorgfältig dosiert werden muss und besonderen Qualitätssicherungs- und Gesundheitsaspekten unterliegt, von großem Vorteil.

Die Handhabbarkeit des erfindungsgemäßen Speicherelements kann vorteilhaft verbessert werden, wenn das tränkbare Material in einem Teilbereich seiner Oberfläche mit einer im Wesentlichen flüssigkeitsundurchlässigen Hülle versehen ist. Diese Hülle ermöglicht es dem Anwender, das Speicherelement anzufassen, ohne in Kontakt mit der Flüssigkeit zu kommen, mit der das Speicherelement getränkt ist. Es ergibt sich so die Handhabungsfreundlichkeit einer Kartusche, jedoch ohne den Aufwand für deren Herstellung in Kauf nehmen zu müssen. Die flüssigkeitsundurchlässige Hülle kann eine Kunststofffolie sein; mitunter ist es aus Stabilitätsgründen auch vorzuziehen, die flüssigkeitsundurchlässige Hülle als Tiefzieh- oder Spritzgussteil aus Kunststoff mit einer gewissen Eigenstabilität zu fertigen.

Bevorzugt ist das Speicherelement im Wesentlichen stabförmig ausgebildet, so dass es zwei Stirnseiten und eine Mantelfläche aufweist. Die Mantelfläche kann zylindrisch, prismenförmig, quaderförmig und dergleichen mehr ausgebildet sein. Durch die stabförmige Ausbildung ist das Speicherelement dem üblichen Design von elektrischen Zigaretten angepasst, wobei in der Regel eine zylindrische Form das in der elektrischen Zigarette vorhandene Raumangebot optimal nutzen wird. Die Stabform ist besonders leicht vom Anwender zu handhaben, was insbesondere dann Vorteile bietet, wenn die flüssigkeitsundurchlässige Hülle die Mantelfläche des stabförmigen Speicherelements im Wesentlichen überdeckt, während die Stirnseiten frei bleiben. Über die Stirnseiten kann dann das Tränken des tränkbaren Materials sowie das Entnehmen bzw. Verdampfen der Flüssigkeit aus dem Speicherelement erfolgen. Gleichzeitig ist der Anwender vor einem Kontakt mit der Flüssigkeit geschützt, wenn er das stabförmige Speicherelement intuitiv an dessen Mantelfläche ergreift.

Im Rahmen der vorliegenden Erfindung ist es schließlich besonders bevorzugt, wenn das erfindungsgemäße Speicherelement von einem Behältnis umschlossen ist, das aus einem wannenförmigen Kunststoffteil und einer dieses verschließenden Siegelfolie besteht. Dieses Behältnis dient zum Schutz des Speicherelements und der darin gespeicherten Flüssigkeit, garantiert die Echtheit und den Herkunftsweg des Speicherelements und insbesondere der darin gespeicherten Flüssigkeit und kann gleichzeitig als Verkaufsverpackung dienen. Mit einem solchen Behältnis ergänzt, wird das erfindungsgemäße Speicherelement in einem Behältnis zur Verfügung gestellt, wie dies auch für pharmazeutische Produkte, insbesondere Medikamente üblich ist.

Mit einem Verfahren nach der vorliegenden Erfindung wird ein erfindungsgemäßes Speicherelement bereitgestellt, das in einem flüssigkeits- und dampfdichten Behältnis verpackt ist. Dieses Behältnis kann, jedoch muss nicht aus einem wannenförmigen Kunststoffteil und einer dieses verschließenden Siegelfolie bestehen.

Um ein entsprechendes Speicherelement, das mit einer zu verdampfenden Flüssigkeit getränkt ist, als verpackte, auswechselbare, separat handhabbare Einheit bereitzustellen, wird das noch nicht mit Flüssigkeit getränkte Speicherelement in ein noch nicht verschlossenes Behältnis eingelegt und die Flüssigkeit direkt in das Behältnis dosiert. Danach wird das Behältnis geschlossen, so dass das Tränken des tränkbaren Materials wenigstens zum Teil im geschlossenen Behälter erfolgt, indem das tränkbare Material die Flüssigkeit absorbiert.

Es ist also im Rahmen des erfindungsgemäßen Verfahrens weder nötig, das tränkbare Material des Speicherelements separat mit der zu verdampfenden Flüssigkeit zu tränken, bevor das Speicherelement in das Behältnis eingelegt wird, noch ist es notwendig, beim Verfahrensschritt des Tränkens des Speicherelements mit der zu verdampfenden Flüssigkeit abzuwarten, bis sich das Speicherelement mit der gesamten Flüssigkeitsmenge vollgesogen hat. Es genügt vielmehr, einfach die erforderliche Menge an Flüssigkeit in das Behältnis zu dosieren, in dem das Speicherelement enthalten ist oder in das das Speicherelement eingelegt wird, und das Behältnis flüssigkeits- und dampfdicht zu schließen. Das Einsaugen bzw. Tränken des Speicherelements mit Flüssigkeit erfolgt dann im geschlossenen Behältnis von selbst, wobei es auch nicht nachteilig ist, wenn dieser Prozess möglicherweise mehrere Stunden dauert. Die Vorteile der Dosiergenauigkeit, des unproblematischen Handlings des getränkten Speicherelements und der kurzen Prozesszeiten liegen auf der Hand.

Besondere Vorteile bietet das erfindungsgemäße Verfahren, wenn als Behältnis ein wannenförmiges Kunststoffteil mit einer dieses verschließenden Siegelfolie verwendet wird. Denn dies ermöglicht insbesondere die Herstellung von Mehrfachpackungen mit einer Mehrzahl von Speicherelementen, die jeweils als Einzelpackung, mitsamt ihrem Behältnis, von der Mehrfachpackung separiert werden können, wie dies auch bei anderen pharmazeutischen Produkten üblich ist. Darüber hinaus kann die Siegelfolie mit einem Aufdruck versehen werden, insbesondere beim oder nach dem Verschließen des Behälters, der Informationen wie Chargennummer und Mindesthaltbarkeit enthält.

Die bevorzugten Ausgestaltungen des erfindungsgemäßen Speicherelements werden auch im Rahmen des erfindungsgemäßen Verfahrens bevorzugt, d.h. vorzugsweise wird ein Speicherelement verwendet, dessen tränkbares Material in einem Teilbereich seiner Oberfläche mit einer im Wesentlichen flüssigkeitsundurchlässigen Hülle versehen ist. Weiter bevorzugt wird ein Speicherelement verwendet, das im Wesentlichen stabförmig mit zwei Stirnseiten und einer Mantelfläche ausgebildet ist, wobei die flüssigkeitsundurchlässige Hülle die Mantelfläche im Wesentlichen überdeckt, während die Stirnseiten frei bleiben.

Im Rahmen der vorliegenden Erfindung wird schließlich ein elektrisches Inhalationsgerät, insbesondere eine elektrische Zigarette vorgeschlagen, die eine elektrische Energiequelle, einen Verdampfer für Flüssigkeiten, ein Speicherelement für die zu verdampfenden Flüssigkeiten und ein Mundstück umfasst und durch das erfindungsgemäße Speicherelement gekennzeichnet ist.

Ausführungsbeispiele für ein erfindungsgemäß ausgestaltetes Speicherelement, das mit einem erfindungsgemäßen Verfahren bereitgestellt wird, sowie ein Ausführungsbeispiel für eine elektrische Zigarette sind im Folgenden anhand der beigefügten Zeichnungen näher beschrieben und erläutert. Es zeigen:
- Figur 1: eine seitliche Schnittdarstellung eines erfindungsgemäß ausgebildeten Speicherelements;
- Figur 2: drei Draufsichten auf Stirnseiten dreier verschiedener Speicherelemente mit unterschiedlichen Querschnitten;
- Figur 3: eine seitliche Schnittdarstellung eines wesentlichen Verfahrensschritts beim Bereitstellen eines erfindungsgemäßen Speicherelements;
- Figur 4: eine seitliche Ansicht eines verpackten Speicherelements;
- Figur 5: eine Draufsicht auf das verpackte Speicherelement gemäß Figur 4;
- Figur 6: eine seitliche Ansicht eines anderen Ausführungsbeispiels für ein verpacktes Speicherelement;
- Figur 7: eine Draufsicht auf das verpackte Speicherelement gemäß Figur 6;
- Figur 8: eine schematische perspektivische Darstellung einer elektrischen Zigarette.

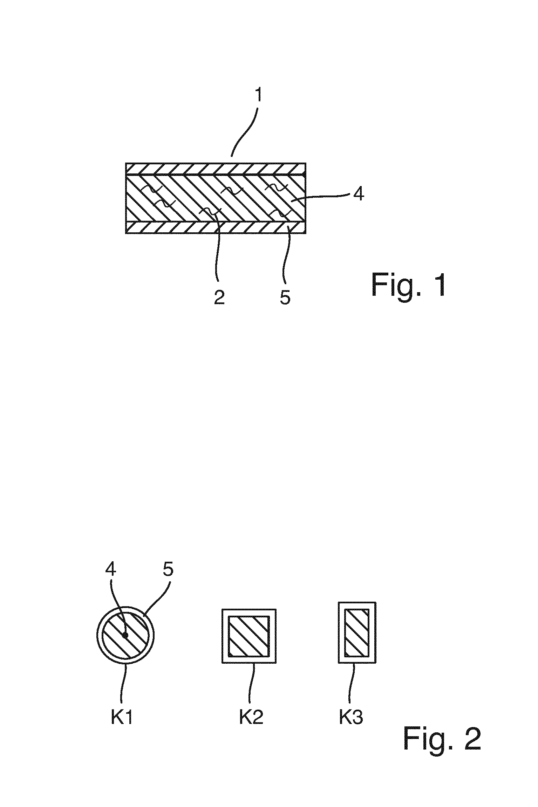

Figur 1 zeigt ein Ausführungsbeispiel für ein erfindungsgemäß ausgestaltetes Speicherelement 1 in einer schematischen seitlichen Schnittdarstellung. Dieses Speicherelement 1 enthält zu verdampfende Flüssigkeit 2 mit einem Wirkstoff, vorliegend Nikotin. Das Speicherelement 1 besteht im Wesentlichen aus einem tränkbaren Material 4, vorliegend aus einem stabförmigen, saugfähigen Gebilde aus gepressten Baumwollfasern, die mit der Flüssigkeit 2 getränkt sind. Die Mantelfläche des stabförmigen Gebildes ist mit einer dünnen Kunststofffolie als flüssigkeitsundurchlässige Hülle 5 umgeben, die in ihren Endbereichen offen ist und so die Stirnseiten 7 des Speicherelements 1 freilässt.

Aufgrund der flüssigkeitsundurchlässigen Hülle 5 kann das Speicherelement 1, dessen tränkbares Material 4 vollständig mit zu verdampfender Flüssigkeit 2 beladen ist, durch Anfassen an der Mantelfläche gehandhabt werden, ohne dass die Finger mit der Flüssigkeit 2 und dem darin gelösten Nikotin in Berührung kommen. Das in Figur 1 dargestellte Speicherelement 1 ist somit ein leicht handhabbarer Wirkstoffstick zum Einsetzen in eine elektrische Zigarette, wobei der Wirkstoffstick leicht ausgewechselt werden kann.

Figur 2 zeigt Beispiele für den Querschnitt des in Figur 1 dargestellten stabförmigen Speicherelements 1: Der Querschnitt kann beispielsweise kreisrund sein (K1), oder quadratisch (K2), oder aber rechteckig (K3). Daneben sind viele andere Querschnittsformen denkbar, deren Geeignetheit im Wesentlichen von den Gegebenheiten innerhalb der elektrischen Zigarette, in die das Speicherelement 1 eingesetzt werden soll, abhängen.

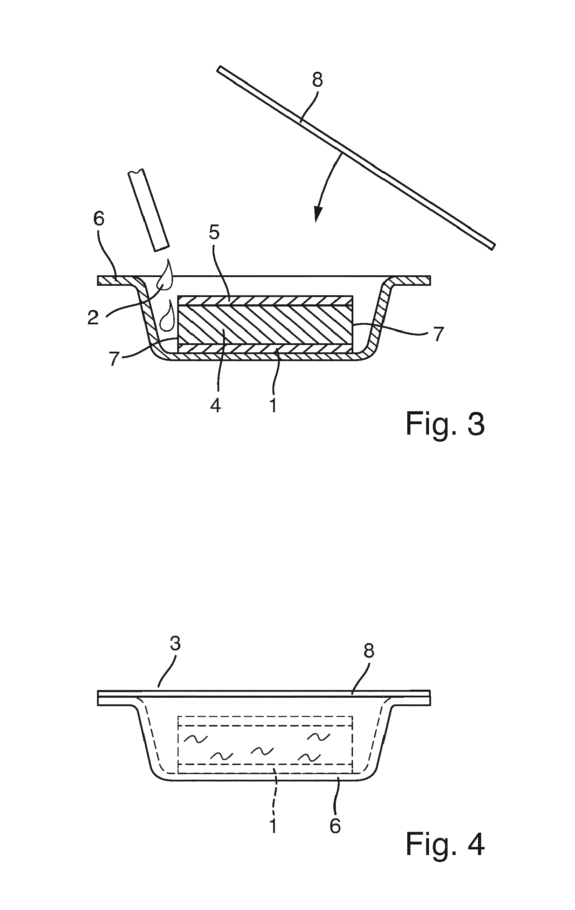

Figur 3 zeigt einen erfindungswesentlichen Moment beim Durchführen des erfindungsgemäßen Verfahrens: Das Speicherelement 1, das wie in Figur 1 aus einem stabförmig gepressten, tränkbaren Material 4 und einer flüssigkeitsundurchlässigen Hülle 5 besteht, die das tränkbare Material 4 an dessen Mantelfläche umgibt und die Stirnseiten 7 freilässt, ist vorliegend noch nicht mit einer zu verdampfenden Flüssigkeit 2 versehen. Das tränkbare Material 4 muss vielmehr noch mit der Flüssigkeit 2 getränkt werden.

Das noch nicht getränkte Speicherelement 1 ist in ein wannenförmiges Kunststoffteil 6 eingelegt worden, das beispielsweise im Tiefziehverfahren oder im Spritzgussverfahren hergestellt worden ist. Zuvor, gleichzeitig oder danach wird eine bestimmte Menge an Flüssigkeit 2 in das wannenförmige Kunststoffteil 6 dosiert, die vom tränkbaren Material 4 des Speicherelements 1 aufgenommen werden kann. Sodann wird das wannenförmige Kunststoffteil 6 mit einer Siegelfolie 8 flüssigkeits- und dampfdicht verschlossen, ohne abzuwarten, bis sich das tränkbare Material 4 des Speicherelements 1 mit der Flüssigkeit 2 vollgesogen hat. Dies kann dann noch anschließend im Lager oder auf dem Vertriebsweg innerhalb des geschlossenen, aus wannenförmigem Kunststoffteil 6 und Siegelfolie 8 gebildeten Behältnisses 3 erfolgen. Die Saugfähigkeit des hier verwendeten tränkbaren Materials 4 sorgt dafür, dass die zu verdampfende Flüssigkeit 2, die in das wannenförmige Kunststoffteil 6 dosiert wurde, vollständig vom Speicherelement 1 aufgenommen wird. Dies erfolgt im Wesentlichen über die offenen Stirnseiten 7 des Speicherelements. Gegebenenfalls kann zur Unterstützung die flüssigkeitsundurchlässige Hülle 5 beispielsweise mit Perforationen versehen sein, um Bereichsweise doch flüssigkeitsdurchlässig zu werden und die Flüssigkeitsaufnahme des tränkbaren Materials 4 auch an der Mantelfläche zu ermöglichen.

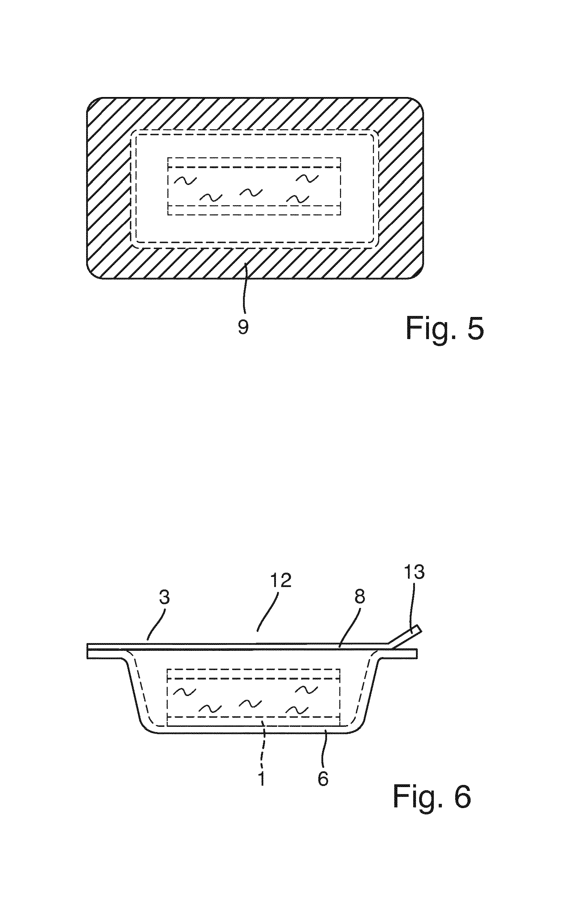

Das Behältnis 3, das aus dem wannenförmigen Kunststoffteil 6 und der Siegelfolie 8, die beispielsweise eine Aluminiumfolie sein kann, besteht, bildet gleichzeitig die Transport- und Verkaufsverpackung für das Speicherelement 1, wie in Figur 4 in seitlicher Ansicht und in Figur 5 in Draufsicht dargestellt ist. Mit durchbrochenen Linien ist jeweils das "Innenleben" des Behältnisses 3 angedeutet.

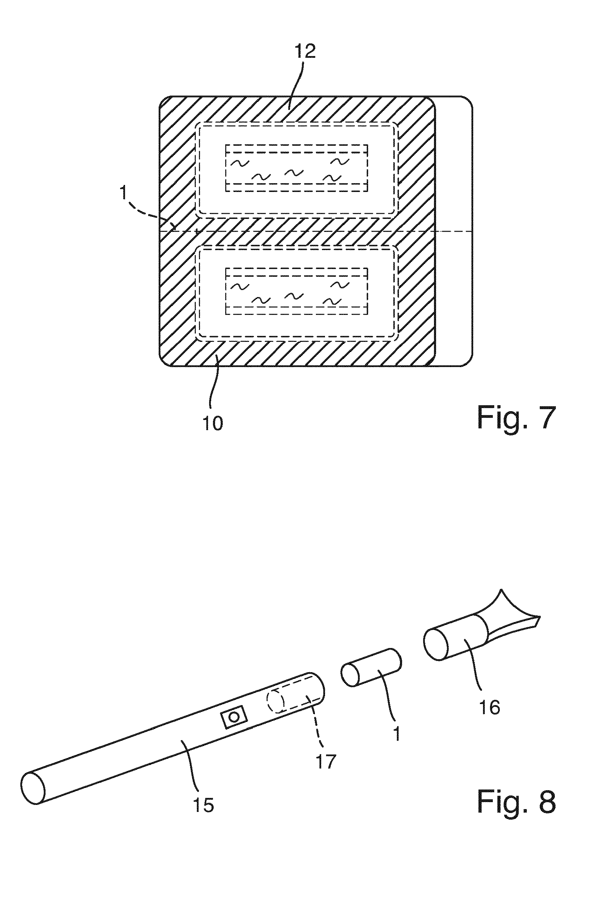

Die Siegelfolie 8 ist plan auf einer breit auskragenden Siegelfläche 9 des wannenförmigen Kunststoffteils 6 aufgebracht und stoffschlüssig mit dieser verbunden. Zum Öffnen des Behältnisses 3 kann die Siegelfolie 8 mit den Fingern oder durch das Speicherelement 1 durchstoßen werden, um das Speicherelement 1 zu entnehmen. Das Behältnis 3 kann jedoch auch als Peelpackung 12 ausgestaltet sein, wie dies die Figuren 6 und 7 zeigen. Hier ist die Siegelfolie 8 in einem jeweils rechts dargestellten Bereich nicht mit der Siegelfläche 9 des wannenförmigen Kunststoffteils 6 verbunden. So bildet sich eine Peellasche 13 zum Abziehen der Siegelfolie 8 vom wannenförmigen Kunststoffteil 6. Diese Ausführungsform eignet sich für Siegelfolien 8, die nicht leicht zerstörbar sein sollen.

Wie Figur 7 außerdem verdeutlicht, kann das vorliegende Behältnis 3, das aus einem wannenförmigen Kunststoffteil 6 und einer Siegelfolie 8 besteht, als Mehrfachpackung 10 gefertigt und befüllt werden. Eine Perforation 11 ermöglicht, die einzelnen Speicherelemente 1 mitsamt dem zugehörigen Einzelbehältnis 3 von der Mehrfachpackung 10 zu separieren, ohne die weiteren Einzelbehältnisse zu beeinträchtigen.

Wie Figur 8 in einer schematischen perspektivischen Darstellung visualisiert, kann das Speicherelement 1 nach seiner Entnahme aus dem Behältnis 3 in eine elektrische Zigarette eingesetzt werden, welche im Wesentlichen aus einer elektrischen Energiequelle 15, einem Verdampfer für Flüssigkeiten 17, dem einzusetzenden Speicherelement 1 und einem Mundstück 16 besteht. Der Verdampfer 17 wird beim Aufsetzen des Mundstücks 16 in Kontakt mit dem Speicherelement 1 gebracht, wodurch die im Speicherelement 1 enthaltene Flüssigkeit verdampft werden kann. Zum Aktivieren des Verdampfers 17 wird ein Schalter 18 betätigt. Gleichzeitig zieht der Anwender am Mundstück 16, so dass Luft durch stromaufwärts des Verdampfers 17 angeordnete Luftöffnungen 19 in das Innere der elektrischen Zigarette gezogen, über den Verdampfer 17 in das Mundstück 16 und von dort zum Anwender geleitet wird. Im Verdampfer 17 nimmt die Luft verdampfte und fein vernebelte Flüssigkeit aus dem Speicherelement 1 auf, so dass der Anwender diese zusammen mit dem darin gelösten Wirkstoff inhalieren kann.

Wenn das tränkbare Material 4 des Speicherelements 1 die darin gespeicherte Flüssigkeit 2 an den Verdampfer 17 abgegeben hat, muss die elektrische Zigarette neu beladen werden. Hierzu wird ganz einfach das verbrauchte Speicherelement 1 aus der elektrischen Zigarette entnommen und ein frisches Speicherelement in die elektrische Zigarette eingesetzt.

Die vorliegende Erfindung ist nicht auf eine elektrische Zigarette bzw. auf eine Verwendung in einer elektrischen Zigarette beschränkt, Sie umfasst vielmehr alle Arten von elektrischen Inhalationsgeräten, um beispielsweise zu inhalierende Medikamente, wie Asthmamittel, Antibiotika gegen Atemwegserkrankungen und dergleichen zu applizieren.

## Patentansprüche

1. Speicherelement für in einem elektrischen Inhalationsgerät, insbesondere in einer elektrischen Zigarette (15, 16, 17) zu verdampfende Flüssigkeiten (2),
**dadurch gekennzeichnet,**
**dass** das Speicherelement (1) im Wesentlichen aus einem Material (4) besteht, das mit Flüssigkeit (2) getränkt werden kann, und als auswechselbare, separat handhabbare Einheit ausgebildet ist.

2. Speicherelement nach Anspruch 1,
**dadurch gekennzeichnet,**
**dass** das tränkbare Material (4) in einem Teilbereich seiner Oberfläche mit einer im Wesentlichen flüssigkeitsundurchlässigen Hülle (5) versehen ist.

3. Speicherelement nach Anspruch 2,
**dadurch gekennzeichnet,**
**dass** das Speicherelement (1) im Wesentlichen stabförmig mit zwei Stirnseiten (7) und einer Mantelfläche ausgebildet ist, wobei die flüssigkeitsundurchlässige Hülle (5) die Mantelfläche im Wesentlichen überdeckt, während die Stirnseiten (7) nicht von der flüssigkeitsundurchlässigen Hülle (5) überdeckt sind.

4. Speicherelement nach mindestens einem der Ansprüche 1 bis 3,
**dadurch gekennzeichnet,**
**dass** das Speicherelement (1) von einem Behältnis (3) umschlossen ist, das aus einem wannenförmigen Kunststoffteil (6) und einer dieses verschließenden Siegelfolie (8) besteht.

5. Verfahren zum Bereitstellen eines Speicherelements (1) für in einem elektrischen Inhalationsgerät, insbesondere in einer elektrischen Zigarette (15, 16, 17) zu verdampfende Flüssigkeiten (2), **gekennzeichnet durch** die Verfahrensschritte:
- Verwenden eines Speicherelements (1), das im Wesentlichen aus einem Material (4) besteht, das mit Flüssigkeit (2) getränkt werden kann,
- Tränken des Speicherelements (1) mit der Flüssigkeit (2),
- Verpacken des Speicherelements (1) in einem flüssigkeits- und dampfdichten Behältnis (3),
- wobei das Speicherelement (1) in das Behältnis (3) eingelegt, die Flüssigkeit (2) in das Behältnis (3) dosiert und das Behältnis (3) dann geschlossen wird, so dass das Tränken des tränkbaren Materials (4) wenigstens zum Teil im geschlossenen Behältnis (3) erfolgt.

6. Verfahren nach Anspruch 5,
wobei ein Behältnis (3) verwendet wird, das aus einem wannenförmigen Kunststoffteil (6) und einer dieses verschließenden Siegelfolie (8) besteht, und wobei das Speicherelement (1) in das wannenförmige Kunststoffteil (6) eingelegt, die Flüssigkeit (2) in das wannenförmige Kunststoffteil (6) dosiert und das wannenförmige Kunststoffteil (6) dann mit der Siegelfolie (8) verschlossen wird.

7. Verfahren nach einem der Ansprüche 5 oder 6,
**dadurch gekennzeichnet,**
**dass** ein Speicherelement (1) nach einem der Ansprüche 2 oder 3 verwendet wird.

8. Elektrisches Inhalationsgerät, umfassend eine elektrische Energiequelle (15), einen Verdampfer (17) für Flüssigkeiten (2), ein Speicherelement (1) für die zu verdampfenden Flüssigkeiten (2) und ein Mundstück (16),
**dadurch gekennzeichnet,**
**dass** das Speicherelement (1) im Wesentlichen aus einem Material (4) besteht, das mit Flüssigkeit (2) getränkt werden kann, und als auswechselbare, separat handhabbare Einheit ausgebildet ist.

9. Elektrisches Inhalationsgerät nach Anspruch 8,
**dadurch gekennzeichnet,**
**dass** das tränkbare Material (4) des Speicherelements (1) in einem Teilbereich seiner Oberfläche mit einer im Wesentlichen flüssigkeitsundurchlässigen Hülle (5) versehen ist.

10. Elektrisches Inhalationsgerät nach Anspruch 9,
**dadurch gekennzeichnet,**
**dass** das Speicherelement (1) im Wesentlichen stabförmig mit zwei Stirnseiten (7) und einer Mantelfläche ausgebildet ist, wobei die flüssigkeitsundurchlässige Hülle (5) die Mantelfläche im Wesentlichen überdeckt, während die Stirnseiten (7) nicht von der flüssigkeitsundurchlässigen Hülle (5) überdeckt sind.

11. Elektrisches Inhalationsgerät nach mindestens einem der Ansprüche 8 bis 10,
**dadurch gekennzeichnet,**
**dass** es als elektrische Zigarette ausgebildet ist.
